# EUROPEAN PATENT SPECIFICATION

(11) **EP 2 818 116 B1**
(45) Date of publication and mention of the grant of the patent: **06.04.2016**
(21) Application number: 14164309.8
(22) Date of filing: 10.04.2014
(51) Int. Cl.: A61B 8/00, A61B 8/08, A61B 6/00

(54) **Computer-assisted ultrasound navigation system for providing assistance during diagnostic and therapeutic operations**
Computergestütztes Ultraschallnavigationssystem zur Bereitstellung von Unterstützung während diagnostischer und therapeutischer Operationen
Système de navigation à ultrasons assistée par ordinateur pour fournir une assistance lors d'opérations diagnostiques et thérapeutiques

(30) Priority: 26.06.2013 IT TO20130527
(43) Date of publication of application: 31.12.2014
(73) Proprietor: MASMEC S.p.A., Modugno (IT)
(72) Inventor: Larizza, Pietro, 70026 Modugno (IT); Vinci, Angelo Michele, 70026 Modugno (IT)
(74) Representative: Bongiovanni, Simone

(56) References cited:
- US-A1- 2006 036 170
- US-A1- 2010 208 963
- US-A1- 2010 298 704
- US-A1- 2013 144 135
- WEIN W ET AL: "Automatic CT-ultrasound registration for diagnostic imaging and image-guided intervention", MEDICAL IMAGE ANALYSIS, OXFORD UNIVERSITY PRESS, OXOFRD, GB, vol. 12, no. 5, 2 October 2008 (2008-10-02), pages 577-585, XP023783020, ISSN: 1361-8415, DOI: 10.1016/J.MEDIA.2008.06.006 [retrieved on 2008-06-19]

## Description

### TECHNICAL FIELD OF INVENTION

The present invention relates to a computer-assisted ultrasound navigation system, in particular for assisting medical personnel during diagnostic and/or therapeutic operations.

### STATE OF THE ART

As is known, ultrasound imaging systems enable obtaining images of a patient's internal organs without any risk (even potential) to the patient and the operator of the imaging system, as the waves striking the human body are absolutely innocuous due to their low frequency (in particular, ionizing radiation is not generated). However, ultrasound images are usually not very comprehensible and are of low resolution.

To obtain higher resolution, it is necessary to use a radiation of higher frequency, such as X-rays, which are used in computerized tomography or CT systems. According to some authors, the (ionizing) radiation employed could be potentially hazardous; for this reason, CT systems cannot be used for long periods on the same subject.

A normal CT actually releases several tens of mSv, while a full-body TAC can release up to 100mSv (according to radiation protection regulations in the workplace, an operator must not be exposed to more than 20mSv of ionizing radiation per annum).

Accordingly, there is a need for providing a system that integrates information arriving in real time from an ultrasound imaging system with more detailed images of the same anatomic region, previously taken by means of a computerized tomography system or a magnetic resonance MR system.

There are several systems on the market (for example the MyLab system produced by ESAOTE) that are capable of integrating images obtained from an ultrasound imaging system with images taken by means of a computerized tomography system. US 2013/0144135 describes a prior art system capable of integrating images obtained from an ultrasound imaging system with images taken by means of a computerized topography system.

### SUBJECT AND ABSTRACT OF THE INVENTION

The object of the present invention is that of providing a system that improves the effectiveness of merging ultrasound /CT images with respect to that provided for by the known art by providing a spatial and volumetric calibration method.

According to the present invention, a system and a method of computer-assisted ultrasound navigation are provided, in particular for assisting medical personnel during diagnostic and/or therapeutic operations, as defined in the appended claims.

### BRIEF DESCRIPTION OF DRAWINGS

- Figure 1 schematically shows a computer-assisted ultrasound navigation system, in particular for providing assistance to medical personnel during diagnostic and/or therapeutic operations, according to a first embodiment;
- Figure 2 schematically shows a computer-assisted ultrasound navigation system, in particular for providing assistance to medical personnel during diagnostic and/or therapeutic operations, according to a second embodiment;
- Figure 3 illustrates a series of operations performed by the ultrasound navigation system according to the present invention; and
- Figure 4 illustrates a detail of the system in Figures 1 and 2.

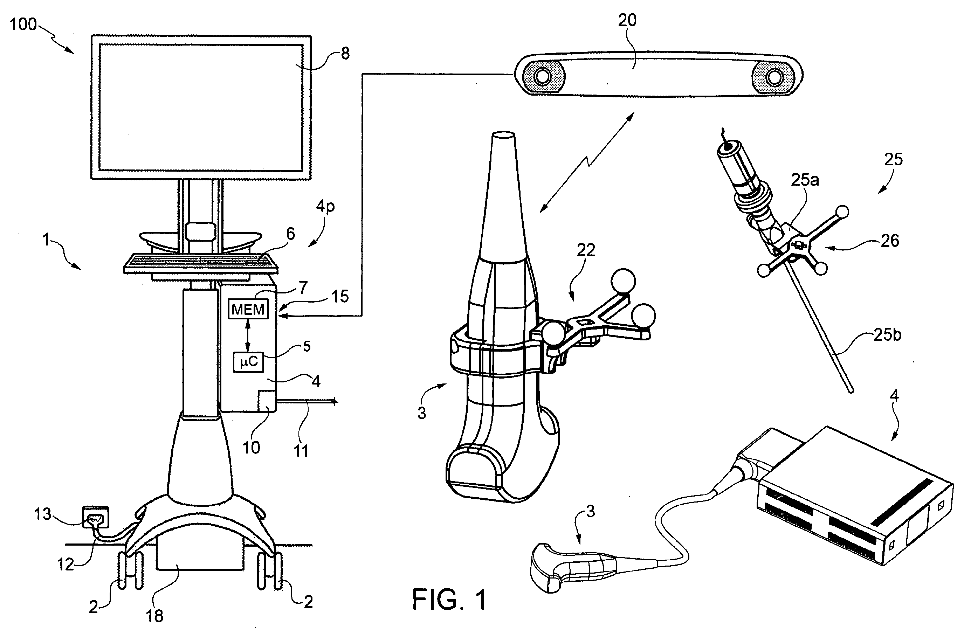

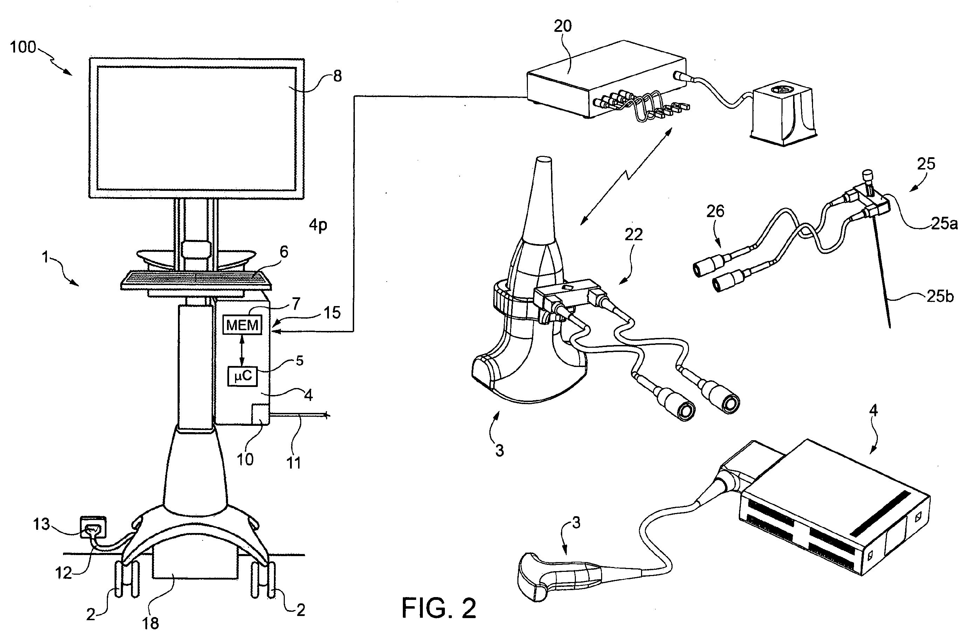

Figures 1 and 2 show a computer-assisted ultrasound navigation system, in particular for assisting medical personnel during diagnostic and/or therapeutic operations 100 embodied according to the present invention. The system 1 comprises an instrumental station 1 and an infrared (Figure 1) or electromagnet (Figure 2) tracking sensor 20, of known type, configured to cooperate with the instrumental station 1. An ultrasound probe 3 operates with an ultrasound device 4 interfaced with the instrumental station 1; the ultrasound probe 3 is of the hand-held type and is combined with a sensor 22, provided with first infrared or electromagnetic marker elements M-I-U or M-E-U to cooperate with the infrared or electromagnetic tracking sensor 20 (as better described hereinafter).

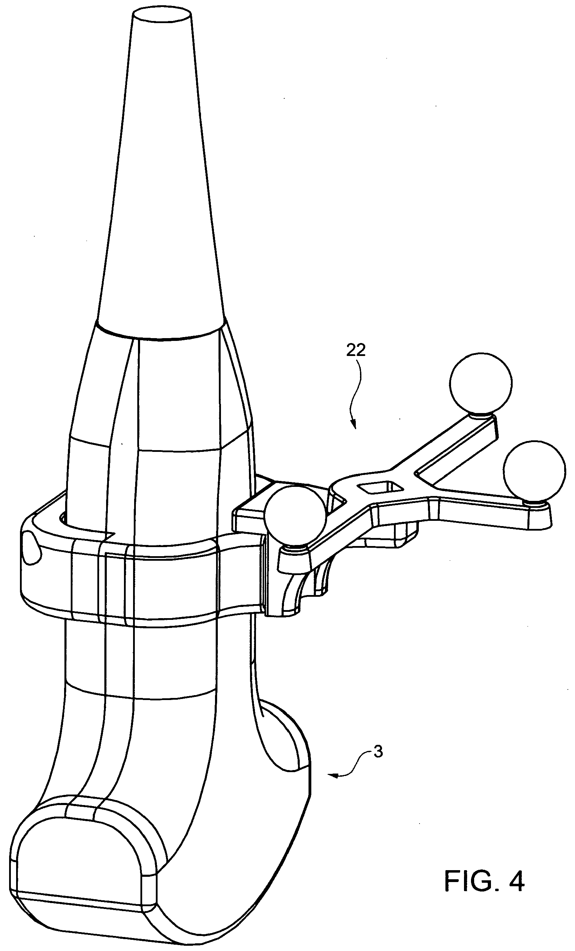

With special reference to Figure 4, the sensor 22 comprises an annular frame suitable for housing a central portion of the probe 3 and a mount extending radially from the annular frame. The mount carries first and second mutually aligned straight arms and a third arm perpendicular to the first two and having a shorter length than that of the first two. The end of each arm carries a spherical body that reflects infrared radiation or, in the case of the electromagnetic version, the annular frame is provided with two electromagnetic sensors (markers).

A surgical (or diagnostic) instrument 25 is provided with second infrared or electromagnetic instrument M-I-S or M-E-U marker elements 26 to cooperate with the infrared/electromagnetic tracking sensor 20 (as better described hereinafter).

In particular, the surgical instrument 25 (an operating tool in the example in Figure 1 and an endoscopic tool in the example in Figure 2) comprises a hand-held proximal portion 25a and an operative distal portion 25b; the marker instrument 26 is coupled to an intermediate portion, leaving the distal portion 25b free. It should be noted that, for greater clarity of representation, the relative sizes of the elements shown in Figures 1 and 2 are not proportional to each other.

The instrumental station 1 (Figures 1 and 2) comprises a processing unit 4p, for example a computer of known type provided with a microcontroller 5 and memory 7, connected to each other; a data input user interface 6, for example including a keyboard and mouse; a display interface 8, for example a high-resolution LCD monitor; and a network interface 10, configured to support a private ad/or public network connection 11 (for example, an Ethernet network).

The instrumental station 1 further comprises a power connection 12, configured to provide the instrumental station 1 with electrical power by means of a wall-mounted power socket 13; a tracking input 15, configured to support a connection 17 (indifferently of the wireless or wire type) between the instrumental station 1, the infrared or electromagnetic tracking sensor 20 and the ultrasound device 4; and an energy storage unit 18, for example a battery, connected to the wall socket 13 by the power connection 12 and configured to temporarily power the instrumental station 1 in the event of an outage in the power provided by the wall socket 13.

According to the embodiment shown, the processing unit 4p is a personal computer (PC), comprising an external protective casing housed on a shelf of the instrumental station 1 and integral with the instrumental station 1 during any movement of the latter on the wheels 2.

The personal computer 4p comprises code instructions (software, described in detail hereinafter) based on algorithms for combining images that are configured to merge information arriving in real time from the ultrasound probe 3 with images previously taken (and therefore stored) by a computerized tomography (CT) or magnetic resonance (MR) diagnostic system. The result of the processing provided by this software is an image that merges the ultrasound image taken in real time with the stored images that have been adapted to the ultrasound image, instant by instant.

In the electromagnetic version (Figure 2) or infrared version (Figure 1), the above-indicated markers perform the role of both rotation and displacement identifiers of the related elements integral therewith (ultrasound probe 3 and surgical instrument 26).

The following description is therefore indifferent to whether the version considered is the one using infrared radiation or the one using electromagnetic waves.

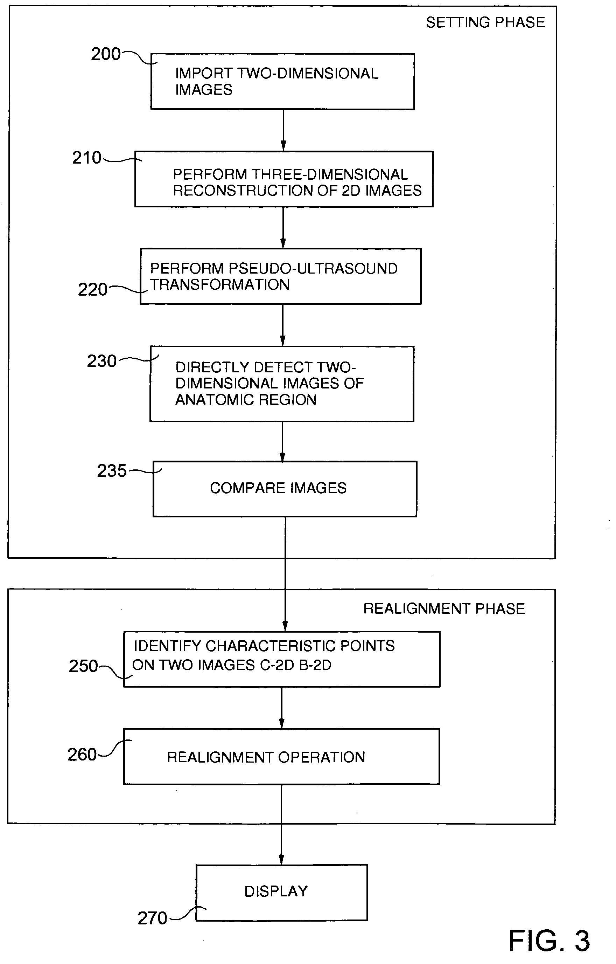

The operations of the system 100 will now be described with reference to the flow chart in Figure 3.

Initially, this starts with a block 200 in which a plurality of two-dimensional images of a patient, obtained by X-ray or magnetic resonance techniques, are imported into the memory 7. The importing can be implemented using a network cable connected to a USB or Ethernet socket of the personal computer 4p or by using computer media, such as a CD-ROM for example.

The two-dimensional images are preferably in the DICOM (Digital Imaging and COmmunications in Medicine) format that - as is known - defines the criteria for the communication, display, filing and printing of biomedical information, such as X-ray images for example. The DICOM standard is public, in the sense that its definition is accessible to everyone. Its diffusion turns out to be extremely advantageous because it enables having a solid information interchange base between apparatuses from different manufacturers, servers and PCs, specific for the biomedical environment.

The imported two-dimensional images regard a complete scan of a concerned anatomic region (for example, the abdomen).

Block 200 is followed by block 210, which starting from the imported two-dimensional images, performs (using known techniques) a very accurate three-dimensional reconstruction (image A) based on scans of the entire anatomic volume of interest.

Block 210 is followed by block 220, which transforms the three-dimensional reconstruction (A) processed by the block 210 into a three-dimensional pseudo ultrasound image (image B) using known methods [1], [2], [3] and [4].
[1] W. Wein et al., Automatic CT-Ultrasound Registration for Diagnostic Imaging and Image-guided Intervention, Medical Image Analysis 12(5), pp 577-585, October 2008
[2] J. L. Dillenseger et al., Fast simulation of ultrasound images from a CT volume, Computers in Biology and Medicine 2009
[3] P. Leroy et al., Rigid Registration of Freehand 3D Ultrasound and CT-Scan Kidney Images, International Journal of Computer Assisted Radiology and Surgery 2007
[4] S. Gill et al., Group-wise registration of ultrasound to CT images of human vertebrae, Medical Imaging 2009

The three-dimensional images A and B refer to the same reference system.

The three-dimensional pseudo ultrasound image is formed by a plurality of two-dimensional pseudo ultrasound images (B-2D). Subsequently, two-dimensional ultrasound images are taken directly of the patient by means of the ultrasound device 4.

The presence of the infrared or electromagnetic sensor 22 coupled to the ultrasound probe enables detecting the position of the sensor in the reference system of the tracking sensor 20 and therefore to orient the captured ultrasound image (image C-2D) in space.

The captured two-dimensional ultrasound image is selected and stored in the memory of the processing unit, together with the spatial coordinates that define it within the reference system of the viewer 20.

Subsequently, processing passes from block 230 to the following block 235, which performs an image comparison operation in order to find the two-dimensional pseudo ultrasound image (image B-2D) that is most similar to the previously stored ultrasound image (C-2D).

The comparison and the search for similarity between the B-2D and C-2D images is carried out by a medical operator in virtue of his/her experience or by means of known algorithms based on correlation methods.

The pseudo ultrasound image B-2D selected by the comparison operations is also stored with coordinates defined in the DICOM domain.

The next step of alignment, or adjustment, starts with block 250, which follows block 235.

In block 250, an operator manually selects at least three characteristic points on the two-dimensional ultrasound image C-2D taken of the patient by means of the ultrasound probe and previously stored and identifies the corresponding characteristic points on the two-dimensional pseudo image U-S B-2D, this also previously stored after the selection operations of block 235.

The identification of the characteristic points can be based on the operator's experience, in this way rendering the processing system capable of recognising shapes of known type and identifying regions of interest in the image, for example having high contrast with respect to adjacent areas or regular and differentiated shapes with respect to adjacent areas.

Block 250 is followed by block 260, in which a realignment operation is carried out between the reference system of the three-dimensional pseudo ultrasound image (image B) and therefore of image A and that of the operative space in which the ultrasound probe operates, in such a way that the identified characteristic points coincide in the respective reference systems of the two two-dimensional images. The realignment or adjustment operation is carried out using roto-translational algorithms known in the literature that - according to the selected characteristic points - calculate a roto-translational matrix that aligns the pseudo ultrasound image reference system (image B) with the reference system of the three-dimensional ultrasound graphic reconstruction regarding the volume associated with the two-dimensional ultrasound images (set of C-2D images) taken of the patient. After block 260, an operative step is performed (block 270 following block 260) in which the three-dimensional reconstruction (image A) processed in block 210 is displayed on the monitor 8.

The following are displayed on the monitor 8 at the same time:
- the two-dimensional ultrasound image acquired in real time by the ultrasound device 4 by means of the ultrasound probe 3; and
- the corresponding MR/TM two-dimensional image belonging to the three-dimensional reconstruction (image A), the respective reference systems being aligned thanks to the alignment or adjustment operation; the images - based on the operations carried out in block 260 and 270 - will thus be perfectly superimposable.

If the surgical instrument 26 is not equipped with sensors (as described above), according to virtual reality methods, it is possible to incorporate the image of the surgical instrument 25 in the two-dimensional image displayed on the monitor 8 based on data coming from the tracking sensor 20 according to the methods set forth in patent EP09425116.

The invention enables having an apparatus that is easy to use for the doctor and oriented to minimally invasive operations, capable of simultaneously displaying the image coming from computerized tomography or magnetic resonance together with the ultrasound image in real time.

In alternative to that described above, the operations in blocks 250 and 260 (realignment step) could comprise the following operations:
A plurality of two-dimensional ultrasound images of the concerned anatomic region (usually the abdomen, but not only) are taken by manually moving the ultrasound probe 3 with respect to the concerned anatomic region.

These two-dimensional images are also displayed in real time on the monitor 8.

The acquired two-dimensional images are processed by means of known ultrasound compound algorithms in order to obtain a very accurate three-dimensional graphic reconstruction (image C).

The presence of an infrared or electromagnetic sensor 22 coupled to the ultrasound probe 3 allows applying a noise reduction algorithm (NRA) in order to form two-dimensional scan planes in a three-dimensional volume with the technique of averaging algorithms.

The operator selects a scan plane P1 on the three-dimensional compound ultrasound image (image C) and identifies one or more characteristic points on this plane P1 (the characteristic points identify an anatomic structure that can be highlighted on the image). A first segmentation procedure is launched that selectively and automatically identifies, on a plurality of scan planes Pn different from that selected (P1), corresponding characteristic points (i.e. points that represent the same anatomic structure on different planes). In this way, a plurality of points Pi is provided that defines the anatomic structure in space on image C.

The operator selects a scan plane P2 on the three-dimensional pseudo ultrasound graphic reconstruction (image B) and identifies similar characteristic points on this plane (the characteristic points identify the aforesaid anatomic structure, but represented in the pseudo ultrasound domain).

A second segmentation procedure is launched that selectively and automatically identifies, on a plurality of scan planes Pk different from that selected (P2), corresponding characteristic points (i.e. points that represent the same anatomic structure on different planes).

In this way, a plurality of points Pj is provided that defines the anatomic structure in space on image B.

The realignment operation is carried out using roto-translational algorithms known in the literature that - based on the first plurality of points Pi and the second plurality of points Pj - calculate a roto-translational matrix that aligns the pseudo ultrasound image reference system (B) with the reference system of the three-dimensional compound ultrasound graphic reconstruction (C).

## Claims

1. A computer-assisted ultrasound navigation system (100) comprising an instrumental unit (1) cooperating with:
- an ultrasound device (4) provided with an ultrasound probe (3) to capture a plurality of two-dimensional ultrasound images of a concerned region;
- an infrared or electromagnetic tracking sensor (20) adapted to detect the position of a sensor (22) coupled to the ultrasound probe and provided with infrared marker elements (M-I-U) or electromagnetic marker elements (M-E-U);
said instrumental unit being configured to detect the position of the sensor in the reference system of the tracking sensor (20) and to orient the captured ultrasound images (C-2D image), the system being **characterized by** implementing:
a) importing means (200) which import a plurality of two-dimensional images, obtained by means of X-ray tomography CT or magnetic resonance MR techniques and related to a full scan of a concerned anatomic region, into the memory (7) of the computer; said two-dimensional images are preferably in DICOM (Digital Imaging and COmmunications in Medicine) format;
b) first reconstruction means (210), which starting from imported two-dimensional images, form a three-dimensional reconstruction of the entire concerned anatomic region by generating a reconstructed three-dimensional image A;
c) transformation means (220), which transform said three-dimensional image A into a three-dimensional pseudo ultrasound image B formed by a plurality of two-dimensional pseudo ultrasound images (B-2D);
d) scanning means (230) by means of which at least one two-dimensional ultrasound image (e) of the concerned anatomic region is captured by manually moving said ultrasound probe (3) with respect to said concerned region; said scanning means (230) being further adapted to detect the position of the sensor (22) coupled to the probe in the reference system of the tracking sensor (20) by orienting the captured two-dimensional ultrasound image in space, which is stored;
e) comparison means (235) configured to extract the two-dimensional pseudo ultrasound image (image B-2D) most similar to said stored ultrasound image (C-2D);
f) selection means (250) of characteristic points on said previously captured, detected and stored two-dimensional ultrasound image (C-2D) and on the extracted two-dimensional pseudo ultrasound image (B-2D);
g) alignment means (250,260) that, according to the selected characteristic points, perform a realignment operation between the reference system of the three-dimensional pseudo ultrasound image (image B) and thus of image A and that of the operative space in which the ultrasound probe operates, so that the identified characteristic points coincide in the respective reference systems of the two two-dimensional images.

2. A system according to claim 1, wherein the characteristic points are identified and selected manually by an operator.

3. A system according to claim 1, wherein the characteristic points are identified and selected by a shape recognition algorithm.

4. A system according to claim 1, wherein operative displaying means (270) are provided in which the following are contextually displayed on a monitor (8) of said computer:
- the two-dimensional ultrasound image acquired in real time by the ultrasound device (4) by means of the ultrasound probe (3); and
- the corresponding MR/TM two-dimensional image belonging to the reconstructed three-dimensional image A; said images - according to said realignment operation - being superimposable.

5. A system according to any of the preceding claims wherein, a surgical instrument is provided with infrared or electromagnetic instrument marker elements to cooperate with the tracking sensor (20);
said computer (100) being configured to display - using virtual reality - the image of the surgical instrument (26) in the two-dimensional image displayed on the monitor (8) according to the data coming from the tracking sensor (20).

6. A system according to claim 1, wherein said realignment means are configured for:
- capturing a plurality of two-dimensional ultrasound images of the concerned anatomic region;
- processing said captured images by means of known ultrasound compound algorithms in order to obtain a three-dimensional graphic reconstruction, known as an ultrasound compound image C;
selecting a scan plane P1 on the three-dimensional compound ultrasound image and identifying one or more characteristic points on such a plane P1;
activating a first segmentation procedure that selectively and automatically identifies corresponding characteristic points on a plurality of scan planes Pn different from the selected scan plane (P1), providing a plurality of points Pi that define the anatomic structure in space on the ultrasound compound image;
selecting a scan plane P2 on the three-dimensional pseudo ultrasound graphic reconstruction (image B) and identifying analogous characteristic points on such a plane P2;
activating a second segmentation procedure that selectively and automatically identifies corresponding characteristic points on a plurality of scan planes Pk different from the selected scan plane (P2), providing a plurality of points Pj that define the anatomic structure in space on the three-dimensional pseudo ultrasound image (B);
using roto-translation algorithms that - according to the first plurality of points Pi and the second plurality of points Pj - calculate a roto-translation matrix that aligns the pseudo ultrasound image reference system (B) with the reference system of the three-dimensional compound ultrasound graphic reconstruction (C).

## Patentansprüche

1. Computergestütztes Ultraschallnavigationssystem (100), das eine Instrumenteneinheit (1) aufweist, die zusammenwirkt mit:
- einer Ultraschallvorrichtung (4), die mit einer Ultraschallsonde (3) versehen ist, um mehrere zweidimensionale Ultraschallbilder einer betroffenen Region aufzunehmen;
- einem Infrarot- oder elektromagnetischen Verfolgungssensor (20), der geeignet ist, die Position eines Sensors (22), der mit der Ultraschallsonde gekoppelt ist und mit Infrarot-Markerelementen (M-I-U) oder elektromagnetischen Markerelementen (M-E-U) versehen ist, zu erfassen;
wobei die Instrumenteneinheit konfiguriert ist, um die Position des Sensors in dem Bezugssystem des Verfolgungssensors (20) zu erfassen und die aufgenommenen Ultraschallbilder (C-2D-Bild) zu orientieren, wobei das System **gekennzeichnet ist durch** Implementieren von:
a) einer Importiereinrichtung (200), die mehrere zweidimensionale Bilder, die mit Hilfe von Röntgenstrahltomografie-(CT-) oder Magnetresonanz-(MR-) Verfahren erhalten werden und sich auf eine vollständige Abtastung einer betroffenen anatomischen Region beziehen, in den Speicher (7) des Rechners importiert; wobei die zweidimensionalen Bilder vorzugsweise in dem DICOM- (Digital Imaging an Communications in Medicine) Format sind;
b) einer ersten Rekonstruktionseinrichtung (210), die beginnend mit importierten zweidimensionalen Bildern eine dreidimensionale Rekonstruktion der gesamten betroffenen anatomischen Region bildet, indem sie ein rekonstruiertes dreidimensionales Bild A erzeugt;
c) einer Transformationseinuchtung (220), die das dreidimensionale Bild A in ein dreidimensionales Pseudo-Ultraschallbild B transformiert, das **durch** mehrere zweidimensionale Pseudo-Ultraschallbilder (B-2D) gebildet wird;
d) einer Abtasteinrichtung (230), mit Hilfe derer wenigstens ein zweidimensionales Ultraschallbild (e) der betroffenen anatomischen Region **durch** manuelles Bewegen der Ultraschallsonde (3) in Bezug auf die betroffene Region aufgenommen wird; wobei die Abtasteinrichtung (230) ferner geeignet ist, die Position des mit der Sonde gekoppelten Sensors (22) in dem Bezugssystem des Verfolgungssensors (20) **durch** Orientieren des aufgenommenen zweidimensionalen Ultraschallbilds in dem gespeicherten Raum zu erfassen;
e) einer Vergleichseinrichtung (235), die konfiguriert ist, um das zweidimensionale Pseudo-Ultraschallbild (Bild B-2D), das dem gespeicherten Ultraschallbild (C-2D) am ähnlichsten ist, zu extrahieren;
f) einer Auswahleinrichtung (250) für charakteristische Punkte auf dem früher aufgenommenen, erfassten und gespeicherten zweidimensionalen Pseudo-Ultraschallbild (Bild C-2D) und auf dem extrahierten zweidimensionalen Pseudo-Ultraschallbild (Bild B-2D);
g) einer Ausrichtungseinrichtung (250, 260), die gemäß den ausgewählten charakteristischen Punkten eine Neuausrichtungsoperation zwischen dem Bezugssystem des dreidimensionalen Pseudo-Ultraschallbilds (Bild B) und somit des Bilds A und dem des Arbeitsraums, in dem die Ultraschallsonde arbeitet, durchführt, so dass die identifizierten charakteristischen Punkte in den jeweiligen Bezugssystemen der zwei zweidimensionalen Bilder zusammenfallen.

2. System nach Anspruch 1, wobei die charakteristischen Punkte von einem Bediener identifiziert und manuell ausgewählt werden.

3. System nach Anspruch 1, wobei die charakteristischen Punkte von einem Formerkennungsalgorithmus identifiziert und ausgewählt werden.

4. System nach Anspruch 1, wobei eine operative Anzeigeeinrichtung (270) bereitgestellt wird, in denen das Folgende kontextabhängig auf einem Monitor (8) des Rechners angezeigt wird:
- das zweidimensionale Ultraschallbild, das von der Ultraschallvorrichtung (4) mittels der Ultraschallsonde (3) in Echtzeit gewonnen wird; und
- das entsprechende zweidimensionale MR/TM-Bild, das zu dem rekonstruierten dreidimensionalen Bild A gehört; wobei diese Bilder - gemäß der Neuausrichtungsoperation - überlagerbar sind.

5. System nach einem der vorhergehenden Ansprüche, wobei ein chirurgisches Instrument mit Infrarot- oder elektromagnetischen Instrumentenmarkerelementen versehen ist, um mit dem Verfolgungssensor (20) zusammen zu wirken;
wobei der Rechner (100) konfiguriert ist, um - unter Verwendung einer virtuellen Realität - das Bild des chirurgischen Instruments (26) in dem zweidimensionalen Bild, das auf dem Monitor (8) angezeigt wird, entsprechend den Daten, die von dem Verfolgungssensor (20) kommen, anzuzeigen.

6. System nach Anspruch 1, wobei die Ausrichtungseinrichtung konfiguriert ist, um:
- mehrere zweidimensionale Pseudo-Ultraschallbilder der betroffenen anatomischen Region aufzunehmen;
- die aufgenommenen Bilder mit Hilfe bekannter Ultraschallzusammensetzungsalgorithmen zu verarbeiten, um eine dreidimensionale graphische Rekonstruktion zu erhalten, die als ein zusammengesetztes Ultraschallbild C bekannt ist;
- eine Abtastebene P1 auf dem dreidimensionalen zusammengesetzten Ultraschallbild auszuwählen und einen oder mehrere charakteristische Punkte auf einer derartigen Ebene P1 zu identifizieren;
- ein erstes Segmentierungsverfahren zu aktivieren, das entsprechende charakteristische Punkte auf mehreren Abtastebenen Pn, die sich von der ausgewählten Abtastebene (P1) unterscheiden, selektiv und automatisch identifiziert, wobei eine Vielzahl von Punkten Pi bereitgestellt wird, die die anatomische Struktur im Raum auf dem zusammengesetzten Ultraschallbild definieren;
- eine Abtastebene P2 auf der dreidimensionalen Pseudo-Ultraschall-Graphikrekonstruktion (Bild B) auszuwählen und analoge charakteristische Punkte auf einer derartigen Ebene P2 zu identifizieren;
- ein zweites Segmentierungsverfahren zu aktivieren, das entsprechende charakteristische Punkte auf mehreren Abtastebenen Pk, die sich von der ausgewählten Abtastebene (P2) unterscheiden, selektiv und automatisch identifiziert, wobei eine Vielzahl von Punkten Pj bereitgestellt wird, die die anatomische Struktur im Raum auf dem dreidimensionalen Pseudo-Ultraschallbild (B) definieren;
- unter Verwendung von Dreh-Translationsalgorithmen, die - gemäß der ersten Vielzahl von Punkten Pi und der zweiten Vielzahl von Punkten Pj - eine Dreh-Translationsmatrix zu berechnen, die das Pseudo-Ultraschallbild-Bezugssystem (B) mit dem Bezugssystem der dreidimensionalen zusammengesetzten Ultraschallgraphikrekonstruktion (C) ausrichtet.

## Revendications

1. Système (100) de navigation à ultrasons assisté par ordinateur comprenant une unité instrumentale (1) coopérant avec :
- un dispositif à ultrasons (4) muni d'une sonde à ultrasons (3) destinée à capturer une pluralité d'images ultrasonores bidimensionnelles d'une région concernée ;
- un capteur de poursuite à ondes infrarouges ou électromagnétiques (20) adapté pour détecter la position d'un capteur (22) couplé à la sonde à ultrasons et muni d'éléments de repérage à ondes infrarouges (M-I-U) ou d'éléments de repérage à ondes électromagnétiques (M-E-U) ;
ladite unité instrumentale étant configurée pour détecter la position du capteur dans le système de référence du capteur de poursuite (20) et pour orienter les images ultrasonores capturées (image C-2D), le système étant **caractérisé par** la mise en oeuvre :
a) de moyens d'importation (200) qui importent dans la mémoire (7) de l'ordinateur une pluralité d'images bidimensionnelles, obtenues par des techniques de tomographie aux rayons X CT ou de résonance magnétique MR et liées à un balayage complet d'une région anatomique concernée ; lesdites images bidimensionnelles étant de préférence au format DICOM (Digital Imaging and COmmunications in Medicine) ;
b) de premiers moyens de reconstruction (210) qui forment, à partir d'images bidimensionnelles importées, une reconstruction tridimensionnelle de toute la région anatomique concernée en générant une image tridimensionnelle reconstruite A ;
c) de moyens de transformation (220) qui transforment ladite image tridimensionnelle A en une image pseudo-ultrasonore tridimensionnelle B formée par une pluralité de images pseudo-ultrasonores bidimensionnelles (B-2D) ;
d) de moyens de balayage (230) au moyen desquels au moins une image ultrasonore bidimensionnelle (e) de la région anatomique concernée est capturée par déplacement manuelle de ladite sonde à ultrasons (3) par rapport à ladite région concernée ; lesdits moyens de balayage (230) étant en outre adaptés pour détecter la position du capteur (22) couplé à la sonde dans le système de référence du capteur de poursuite (20) par orientation dans l'espace de l'image ultrasonore bidimensionnelle capturée qui est mémorisée ;
e) de moyen de comparaison (235) configurés pour extraire l'image pseudo-ultrasonore bidimensionnelle (image B-2D) la plus similaire à ladite image ultrasonore mémorisée (C-2D) ;
f) de moyens de sélection (250) pour sélectionner des points caractéristiques dans ladite image ultrasonore bidimensionnelle (C-2D) précédemment capturée, détectée et mémorisée et sur l'image pseudo-ultrasonore bidimensionnelle extraite (B-2D) ;
g) de moyens d'alignement (250, 260) qui effectuent, en fonction des points caractéristiques sélectionnés, une opération de réalignement entre le système de référence de la image pseudo-ultrasonore tridimensionnelle (image B) et donc de l'image A et celle de l'espace de travail dans lequel la sonde à ultrasons fonctionne de sorte que les points caractéristiques identifiés coïncident dans les systèmes de référence respectifs des deux images bidimensionnelles.

2. Système selon la revendication 1, dans lequel les points caractéristiques sont identifiés et sélectionnés manuellement par un opérateur.

3. Système selon la revendication 1, dans lequel les points caractéristiques sont identifiés et sélectionnés par un algorithme de reconnaissance de formes.

4. Système selon la revendication 1, dans lequel des moyens d'affichage opérants (270) sont prévus dans lesquels les éléments suivants sont contextuellement affichés sur un écran (8) dudit ordinateur :
- l'image ultrasonore bidimensionnelle acquise en temps réel par le dispositif à ultrasons (4) au moyen de la sonde à ultrasons (3) ; et
- l'image bidimensionnelle MR/TM correspondante appartenant à l'image tridimensionnelle reconstituée A ; lesdites images étant superposables en fonction de ladite opération de réalignement.

5. Système selon l'une quelconque des revendications précédentes, dans lequel un instrument chirurgical est muni d'éléments de repérage d'instrument à ondes infrarouges ou électromagnétiques pour coopérer avec le capteur de poursuite (20) ;
ledit ordinateur (100) étant configuré pour afficher, à l'aide d'une réalité virtuelle, l'image de l'instrument chirurgical (26) dans l'image bidimensionnelle affichée sur l'écran (8) en fonction des données provenant du capteur de poursuite (20).

6. Système selon la revendication A, dans lequel lesdits moyens de réalignement sont configurés pour :
- capturer une pluralité d'images ultrasonores bidimensionnelles de la région anatomique concernée ;
- traiter lesdites images capturées à l'aide d'algorithmes connus de composites ultrasonores afin d'obtenir une reconstruction graphique tridimensionnelles, connue sous le nom d'une image ultrasonore composite C ;
- sélectionner un plan de balayage P1 dans l'image composite ultrasonore tridimensionnelle et identifier un ou plusieurs points caractéristiques dans un tel plan P1 ;
- activer une première procédure de segmentation qui identifie de manière sélective et automatique des points caractéristiques correspondants dans une pluralité de plans de balayage Pn différents du plan de balayage sélectionné (P1), produire une pluralité de points Pi qui définissent la structure anatomique dans l'espace dans l'image composite ultrasonore ;
- sélectionner un plan de balayage P2 dans la reconstruction graphique pseudo-ultrasonore tridimensionnelle (image B) et identifier les points caractéristiques analogues dans un tel plan P2 ;
- activer une seconde procédure de segmentation qui identifie de manière sélective et automatique des points caractéristiques correspondants dans une pluralité de plans de balayage Pk différents du plan de balayage sélectionné (P2), produire une pluralité de points Pj qui définissent la structure anatomique dans l'espace de l'image pseudo-ultrasonore tridimensionnelle (B) ;
- utiliser des algorithmes de roto-translation qui calculent, en fonction de la première pluralité de points Pi et de la seconde pluralité de points Pj, une matrice de roto-translation qui aligne le système de référence d'image pseudo-ultrasonore (B) avec le système de référence de la reconstruction graphique composite pseudo-ultrasonore tridimensionnelle (C).
